# EUROPEAN PATENT SPECIFICATION

(11) **EP 3 122 384 B1**
(45) Date of publication and mention of the grant of the patent: **05.12.2018**
(21) Application number: 15722756.2
(22) Date of filing: 24.03.2015
(51) Int. Cl.: A61L 2/04, B27D 1/08, B27J 5/00, B27K 7/00, B65D 39/00, A61L 2/08, A61L 2/12, A61L 2/20, A61L 2/025

(54) **PROCESS FOR MANUFACTURING NATURAL CORK STOPPERS THROUGH SPIRAL WINDING OF PURE CORK LEAF AND CORK OBTAINED IN THIS WAY**
VERFAHREN ZUR HERSTELLUNG VON NATURKORKSTOPPERN DURCH SPIRALWICKELN VON REINEM KORKBLATT UND AUF DIESE WEISE ERHALTENER KORK
PROCÉDÉ DE FABRICATION DE BOUCHONS EN LIÈGE NATUREL PAR ENROULEMENT EN SPIRALE D'UNE FEUILLE DE LIÈGE PUR, ET LIÈGE OBTENU DE CETTE MANIÈRE

(30) Priority: 26.03.2014 CH 467142014
(43) Date of publication of application: 01.02.2017
(73) Proprietor: Bernasconi, Brunello, 6965 Cadro (CH)
(72) Inventor: Bernasconi, Brunello, 6965 Cadro (CH)
(74) Representative: Galassi, Alessandro
(86) International application number: PCT/IB2015/000394
(87) International publication number: WO 2015/145244

(56) References cited:
- WO-A2-2008/113608

## Description

This invention refers to a manufacturing process of natural cork stoppers free from biological contaminating and synthetic products, by spiral winding on itself of a previously treated cork leaf, in a controlled environment.

### State of the Art

The conventional stoppers on the market, excluding the low-cost type built from ground and with glue mixed cork agglomerate, are usually obtained from a cork block preselected in a single piece through punch cutters, the stoppers built in this way are composed of a single piece of cork and subsequently are finished with lapping machines. The subsequent phase includes the sterilization which because of implied features of the process can only reach the surface of the stopper and not the interior, so preventing the full sterilization. The conventional punching implies in addition the impossibility to obtain stoppers all of them with the same desired characteristics.

The conventional stoppers show two main problems.

The trichloroacetic acid (TCA) is a natural component which at e certain level can jeopardize the organoleptic characteristics of the wine, modifying its flavour and aroma. A wine with a high level of TCA is described as a wine which "smacks of the cork".
The wines contain usually TCA at very low levels (2-5 parts of trillions, 0.000000000002-0.000000000005 grams in a litre of wine), therefore measurable or detectable with difficulty.

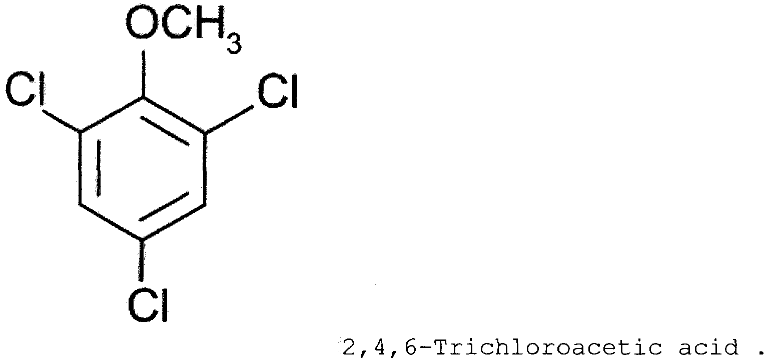

The red powders, composed of the necrotic residues of the layers of material from which the cork originates, are the main cause of the alteration of the organoleptic and aesthetic characteristics of the wine, so resulting indispensable their removal.

The conventional way of manufacturing by means of punching did not succeed in eliminating in full the red powders present inside the stopper, that are responsible for serious damages for the bottled wine: said powders, being contained inside all the hollows (pores) of the cork, are present inside any conventional even first-choice stopper. Such powders can be observed simply sectioning the stopper and cause the transfer to the wine of tannic and organic substances that contribute to the alteration of organoleptic qualities, causing the usually called taste of cork; if we refer only the quality wine produced in Europe such problem causes yearly losses of many billions Euro.

The conventional manufacturing implies in addition high costs, waste and differences in the specifications between a stopper and the other.
The Erriu patent (PCT /EP2008002328 (08716682.3) as well as what is known from the current state of the art have tried to obviate such drawbacks by means of a stopper by spiral winding of cork leafs.

Such method shows from one side the drawback of the incomplete elimination of the red powders with at most only a partial reduction of their presence, and from the other side the addition of the not natural foreign materials to the leaf and to the stopper like glues, films or interposed layers of synthetic material, adhesive tapes, etc..

Furthermore it is not to leave out that, in all the before mentioned methods, the gluing substances or the adhesive materials inserted into the stoppers known till now, observing the food certifications, are as much intolerable from the point of view of the health as slower is the ageing process of such substances. Migrations and direct or indirect contaminations of such substances become problematic and can have serious short and/or long term consequences on the health of the human beings; the field of the adhesive for alimentary use, being at today till now not yet regulated, still shows big risks.

### Innovations brought in by this invention

This invention refers now, unlike the known State of the Art, to a process for the manufacturing and production of stoppers of natural cork, type as you like, starting from fully natural and sterile cork leafs by spiral winding of said leafs as you like on a core or on themselves. The compacting of the stopper by means of the process occurring by catalyzing and activating the natural adhesive waxes contained in the leafs of the cork itself.

Such process allows to obtain cork stoppers of the best quality, aseptic by means of a sterilization phase made possible not only by blowing and/or radiation as in the known state of the art, but by subsequent boiling and hyperbaric treatment at high temperature.

Such process, besides the possibility to pre-select uniform cork leafs, furthermore allows to obtain stoppers having the same specifications of porosity and density as you like according to the product that you like to obtain, also allowing to obtain products aimed to calibrate the exchange oxygen-wine according to the characteristics of the vine-growing and wine-producing product, you like to bottle.

Recent scientific studies have demonstrated that the density and porosity of the cork, two of the characteristics that define the quality of the stoppers, depend on the radial position with respect to the growth of the bark. By the process subject of this invention, unlike the known art, the leaf can be radially laminated allowing to obtain a superior quality stoppers with uniform and pre-determinable density, elasticity module und porosity. So such process allows the wine producers to select stoppers with specific characteristics according to the desired product, also allowing to obtain products aimed to calibrate the exchange oxygen-wine according to the characteristics of the vine-growing and wine-producing product, and/or the distillate or other you like to contain.

Unlike what is described in the previous State of the Art, by the process subject of this invention the leaf can be laminated perpendicularly or parallelly to the growth of the cork, meaning a substantial innovation. These two alternatives change in a significant way the behaviour of the stopper in the bottle, because the attrition changes with the orientation of the microscopic cells of the cork (parallelepipeds from 10 to 40 µm), allowing to pre-determine and obtain the desired characteristics for every stopper and type of glass.

This allows a correct maturity of the wine, being the result of the interaction between the wine and the oxygen present in the atmosphere, unlike the modern oenological practice, which forces the maturity of the wine with different techniques forcing aroma and flavour, stressing sense of smell and sense of taste.

By the process subject of this invention, unlike the processes described by the known technique, we obtain a product free from red powders, with drastic reduction of scrap, implying a noticeable economic saving and low environmental impact by elimination of the waste of the basis material; furthermore with the possibility of orientation of the cutting direction to obtain the desired direction of porosity, full internal sterilization, unlike the grade and levels of sterilization obtained by the methods of the known technique, and pre-determination of the physical characteristics like in particular the density parameters and elasticity module.

Of course the roll obtained by the wound leaf shall at the end remain compact, close and bound and shall not flake off, but unlike the methods of the known technique, neither the method subject of this invention use impregnating or gluing agents added before, during or after the winding, nor bi-adhesive tapes are arranged on the two sides of the leaf to assure the sticking, nor layers of synthetic materials are interposed, like cellophane, like that used by cooking, transparent, heat sealing, nor other mechanical means of seizure.

By the method subject of this invention the compacting of the cork leaf occurs by self-sticking of the leaf layers themselves that originate during its winding by exploiting the natural substances inborn into the cork, precisely by catalyzing the natural adhesive waxes contained inside the cork itself, mainly in the cork oak, ceric oxide and friedeline composing for about the 5% the cellular wall of the cork, corresponding to the following chemical formula.

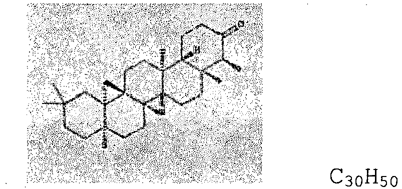

The method described afterwards, subject of this invention, allows to carry out a production process able to obtain stoppers of excellence free from biological contaminators with full removal of the red powders and complete internal sterilization, elimination of scraps, orientation of cutting to set the desired direction of the porosity, and the predetermination as you like of the density and the other physical characteristics.

### Description

This invention refers to a manufacturing and production process of a spiral of leafs of natural cork and to the stopper obtained in this way.

More precisely this invention refers to that is described in the claim 1 and in the claim 6. Particular ways of carrying out this process are described in the claims from 2 to 5.

A natural cork leaf is used preferably of thickness ranging between 0.5 - 1 mm, duly subjected to accurate treatments of cleaning and sterilization with a more significant effectiveness than starting from the classic small square used as punch or by simple winding of the leaf not fully sterilized and anisotropic.

The stopper obtained by the process subject of this invention by means of natural cork leafs, on the contrary, besides to be fully sterile is deprived of any synthetic or plastic component and fully free from any chemical or synthetic not natural adhesive product. We avoid in this way contaminations or migrations of substances alien to the wine, reducing the costs of monitoring adhesives and binders and eliminating the risks coming from the use of adhesive for alimentary use available on the market, currently not fully regulated.

Preferably, on a cylindrical core of natural cork or conical or of another shape suitable for the intended use, the cork leaf is spiral wound without adhesives, because at more than 40% of humidity and at temperature of about 70°C the cork release by nature substances of waxy nature (ceric oxide and friedeline that build about the 5% of the cellular wall of the cork), that serve as natural adhesive for the spiral which, by winding itself, sticks on itself.

We give now in the following a description of an not limiting example of carrying out of the finding subject of this invention with reference to the figures of the enclosed drawings.

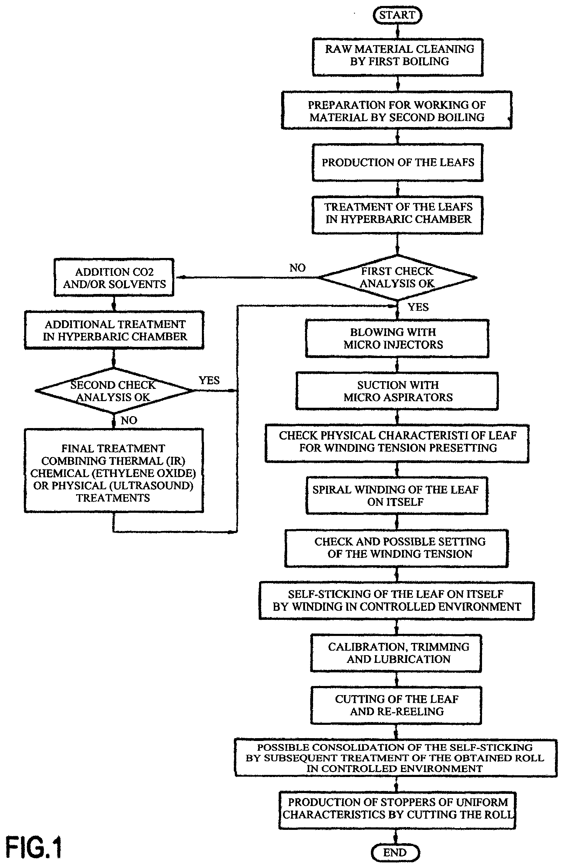

- The Fig.1 shows in a block diagram the process of this invention with reference to the Claim 1.

The subject process includes the following steps:
- First phase of cleaning of the raw material: 60-90 min boiling;
- Second boiling to make the material suitable for the working;
- Preparation of the leaf of thickness preferably from 0,5 mm to 1 mm starting from the plank of cork after removal of the belly and back that is of the part in contact with the trunk of the tree and of the superficial part outside the most outer bark and of a variable width according to the production technology usually from 4 cm to 2 m;

- Treatment in hyperbaric chamber under temperature conditions ranging from 100°C and 150°C, and variable times ranging at a certain humidity from 60% to 90% respectively from 1 and 24 hours, depending on the quantity of contaminating agents, like parasites, moulds, fungus and of 2-4-6 trichloroacetic acid (TCA), chloroanisole produced by the metabolism of the fungus Armillaria mellea, and of water-soluble substances like mineral salts or tannins;
- If from a first check analysis it results that the cork has not reached the required sterilization level we perform an additional treatment repeating the treatment in the hyperbaric chamber with the addition of CO₂ and co-solvents, obtaining a leaf practically fully aseptic, free from every agent and/or contaminating substance, read for the manufacturing phase of the stopper;

- In the case a second check analysis reveals again an incomplete full sterilization, we perform a further final treatment as we like combining thermal (infrared, ..), chemical (ethylene oxide, ..) or physical (ultrasounds, ..) treatments;
- For further safety we finally perform the blowing by means of micro-injectors arranged just before the start of the phase of winding of the cork leaf, able to fully clean the lenticular hollows (lenticels) eliminating possible residues of "red powders" inside said lenticular hollows (lenticels), where in particular the (TCA) 2-4-6 trichloroacetic acid makes a deposit, main responsible of the taste of cork;
- A subsequent micro-suction on the surfaces of the leaf completes the previous cleaning operation before the start of the winding, by suction by means of micro-aspirators arranged on both sides of the leaf with the aim to evacuate possible residues of "red powders" on said sides and inside the hollows of the said leaf.
- The sterilized leaf, free from every agent and/or contaminating substance, ready for the manufacturing phase of the stopper is prepared to be spiral wound on itself or on a core suitably shaped according to the intended use, usually cylindrical, of natural cork on which the cork leaf winds itself as a spiral without addition of chemical agents or adhesive or heat sealing materials;
- In environmental controlled area, without intervention of external glues or adhesives, at more than 40% of humidity and at temperature ranging between 60°C - 80°C, we obtain the release of the waxy substances by nature contained in the cork (ceric oxide and friedeline building about the 5% of the cellular wall of the cork), that act as natural adhesive consequently producing the self-sticking of the cork leaf which by spiral self-winding sticks on itself by extracting said waxy adhesive substances contained in itself;
- Determination, control and achievement of the desired parameters of density, compressibility, porosity and gas permeability of the resulting stopper, by setting the winding tension of the spiral of cork and the winding direction (clockwise or counter-clockwise);
- Final calibration, trimming, lubrication of the end product by vaporization of glycerine, to favour the insertion of the stopper into the neck of the bottle;
- Shearing of the leaf and re-reeling for the manufacturing of the subsequent batch of stoppers;
- Possible strengthening of the self-sticking by subsequent treatment of the obtained roll or of the stoppers manufactured from said roll in controlled environments;
- Obtainment of uniform stoppers of the same characteristics and properties by cutting the roll.

The process in question subject of this invention implies furthermore the possibility to pre-determine density - elasticity and compressibility - porosity and its orientation - dimensions - gas permeability.

By means of the exploitation of a substance present on the cellular wall (ceric oxide / friedeline) in the amount of 5%, under very particular conditions, well studied and calculated, it occurs a self-sticking of the leaf which will build the stopper precisely by catalyzing said substances that duly extracted become adhesive.

### Process 1

A cylindrical core of natural cork on which the cork leaf winds itself as spiral without adhesives because over 40% of humidity at temperature of about 70°C the cork releases by nature waxy substances (ceric oxide and friedeline composing the 5% of the cellular wall of the cork) acting as natural adhesive for the spiral which by self-winding sticks on itself.

### Process 2

According to the process 1, exploiting the working conditions by adjustable vacuum operation the cork leaf is forced to wind on itself and to stick on itself, possibly by clamping its ends.

### Process 3

According to the process 1, at the stated conditions of humidity and temperature of the cork leaf, exploiting the centripetal force, the leaf is forced to wind on itself and to stick on itself up to the building of the stopper.

In all three cases by means of the exploitation of a substance present on the cellular wall (ceric oxide / friedeline) in the amount of 5%, it will be possible, under determined and calculated conditions subject of studies and tests as described before, to trigger the reaction of self-sticking of the leaf which will build the stopper.

Furthermore the processes subject of this invention include the possibility to pre-determine specific weight - elasticity and compressibility - porosity and its orientation - dimensions - gas permeability.

In fact the leaf can be obtained preliminarily by rolling, radially with respect to the growth of the bark, the position with respect to the radius determining the characteristics of density and porosity of the cork, characteristics that mainly determine the quality of the stoppers, allowing to obtain stoppers of superior quality with uniform density, elasticity module and porosity preset as you like.

In addition setting subsequently the tension of the winding, elasticity, density, compressibility, porosity, gas permeability of the obtained stopper are precisely determined.

The final phase includes calibration, trimming, lubrication of the end product by vaporization of a natural lubricant to favour the insertion of the stopper into the neck of the bottle.

The process described up to now remains the same for all the kinds of stoppers produced, wine, champagne, sparkling wine, distillates, spirits.

The produced stopper will be then free from every type of chemical substance and/or adhesives for alimentary use added and used in the current manufacturing of stoppers on the market, because 100% biological.

The process subject of this invention allows to reach remarkable advantages like the full removal of the red powders, the elimination of scrap, the orientation of cutting to obtain the desired direction of the porosity, the full internal sterilization, with preset physical characteristics of density, elasticity and compressibility, producing a stopper for vine-growing and wine-producing products and distillates aimed to overcome the drawbacks currently present on the market.

## Claims

1. Process for production and manufacturing of stoppers of spiral wound cork leaf, **characterized by** the fact that it includes the following operations, consisting of the phases hereby described:
a) First phase of cleaning of the raw material by 60-90 min boiling
b) Second boiling;
c) Preparation of the leaf starting from the plank of cork after removal of the belly and back that is of the part in contact with the trunk of the tree and of the superficial part outside the most outer bark;
d) Treatment in hyperbaric chamber, under temperature conditions ranging between 100°C and 150°C, variable humidity and time respectively ranging between 60% and 90% and between 1 and 24 hours, according to the quantity of contaminating agents, like parasites, moulds, fungus and of 2-4-6 trichloroacetic acid (TCA), chloroanisole produced by the metabolism of the fungus Armillaria mellea, and of water-soluble substances like mineral salts and tannins;
e) Subsequent check analysis of the result obtained by the above treatment described at point d);
f) If from the analyses following the treatment described at point e) it results that the cork is not fully sterilized, we repeat an additional treatment in hyperbaric chamber with the addition of carbon dioxide, substance known for its antibacterial and fungicide properties, and/or in presence of addition of co-solvents;
g) In the case the treatment according to the previous point f) does not result again fully effective highlighting that the cork is not yet fully sterilized, we perform a further final treatment as we like combining thermal (infrared), chemical (ethylene oxide) or physical (ultrasounds) treatments;
h) For further safety we finally perform the blowing by means of micro-injectors arranged just before the start of the phase of winding of the cork leaf, able to fully clean the lenticular hollows (lenticels) eliminating possible residues of "red powders" inside said lenticular hollows (lenticels), where in particular the (TCA) 2-4-6 trichloroacetic acid makes a deposit, main responsible of the taste of cork;
i) Completion of the previous phase h) before the start of the winding phase, by suction by means of micro-aspirators arranged on both sides of the leaf with the aim to evacuate possible residues of "red powders" on said sides and inside the hollows of the said leaf;
j) Performing a check on the fully sterilized leaf, free from every agent and/or contaminating substance for determining its physical characteristics to set the winding tension, to reach the desired parameters of density, compressibility, porosity, gas permeability of the resulting stopper and ready for the manufacturing phase of the stopper, and preparing said leaf to be spiral wound on itself or on a core suitably shaped of natural cork on which the said cork leaf winds itself as a spiral without addition of adhesive or heat sealing materials;
l) Final calibration, trimming, lubrication of the end product by vaporization of glycerine, to favour the insertion of the stopper into the neck of the bottle;
m) Shearing of the leaf and re-reeling for the manufacturing of the subsequent batch of stoppers;
said process being **characterized in that**:
k) In an area with controlled environment, without intervention of external glues or adhesives, at more than 40% of humidity and at temperature ranging between 60°C and 80°C, we obtain the release of the waxy substances by nature contained in the cork (ceric oxide and friedeline building about the 5% of the cellular wall of the cork), that act as natural adhesive consequently producing the self-sticking by catalyzing of said substances of the cork leafs that by spiral self-winding stick on themselves by extracting and transforming said waxy adhesive substances contained in themselves;
and **in that**:
in an environment under adjustable pressure, from 4 to 30 bar, we force through clamping of its ends the sheet or leaf of cork to wind on itself sticking and compacting itself, coming back to the atmospheric pressure, the cork regains its initial natural dimensions, then assuring the desired compactness and airtightness.

2. Process for production and manufacturing of stoppers of spiral wound cork leaf, according to the Claim 1, **characterized by** the fact that exploiting the working conditions by vacuum operation adjustable to the set conditions of humidity and temperature of the cork leaf, we force said leaf through the centripetal force to wind on itself up to the building of the stopper.

3. Process for production and manufacturing of stoppers of spiral wound cork leaf, according to any one of the Claims 1 to 2, **characterized by** the fact that the leaf is obtained preliminarily by rolling, radially with respect to the growth direction of the bark, the position with respect to the radius determining the characteristics of density and porosity of the cork, characteristics that mainly determine the quality of the stoppers, allowing to obtain stoppers of superior quality with uniform density, elasticity module and porosity preset as you like.

4. Process for production and manufacturing of stoppers of spiral wound cork leaf, according to the Claim 3, **characterized by** the fact that the spiral winding core is conical or shaped suitably to the intended use.

5. Process for production and manufacturing of stoppers of spiral wound cork leaf, according to any one of the Claims 1 to 4, by subsequent treatment of the obtained roll or of the stoppers obtained by cutting said roll in a controlled environment strengthening consolidation and self-sticking therefor.

6. Stopper of spiral wound cork leaf, manufactured according the process described in any one of the Claims 1 to 5, **characterized by** the fact that it is free from any synthetic or plastic component and fully free from any chemical or not natural adhesive substance, with preset characteristics of dimensions, elasticity, compressibility, density, porosity, gas permeability.

## Patentansprüche

1. Verfahren zur Produktion und Herstellung von Verschlüssen aus spiralförmig gewickeltem Korkblatt, **gekennzeichnet durch** die Tatsache, dass es die folgenden Vorgänge umfasst, welche aus den hierdurch beschriebenen Phasen bestehen:
a) eine erste Phase eines Reinigens des Rohmaterials **durch** 60-90 minütiges Kochen
b) ein zweites Kochen;
c) eine Vorbereitung des Blattes ausgehend von der Planke des Korks nach einer Entfernung des Bauches und des Rückens, d.h. des Teils, welcher in Berührung mit dem Stamm des Baumes steht und des oberflächigen Teils außerhalb der äußersten Rinde;
d) eine Behandlung in einer Überdruckkammer unter Temperaturbedingungen im Bereich zwischen 100 °C und 150 °C, einer variablen Feuchtigkeit und Zeit im Bereich zwischen 60 % und 90 % bzw. zwischen 1 und 24 Stunden, gemäß der Menge von kontaminierten Mitteln, wie Parasiten, Schimmel, einem Pilz und 2-4-6 Trichloressigsäure (TCA), Chloranisol, welches **durch** den Stoffwechsel des Pilzes Armillaria mellea produziert wird, und von wasserlöslichen Substanzen wie Mineralsalzen und Tanninen;
e) eine anschließende Kontrollanalyse des Ergebnisses, welches **durch** die obige, bei Punkt d) beschriebene Behandlung erhalten wurde;
f) Wenn aus der der Behandlung folgenden Analyse, welche bei e) beschrieben ist, resultiert, dass der Kork nicht vollständig sterilisiert ist, wiederholen wir eine zusätzliche Behandlung in einer Überdruckkammer, mit dem Zusatz von Kohlenstoffdioxid, einer Substanz, welche für ihre antibakteriellen und fungiziden Eigenschaften bekannt ist, und/oder in Gegenwart eines Zusatzes von Hilfslösungsmitteln;
g) in dem Fall, dass die Behandlung gemäß dem vorhergehenden Punkt f) wieder nicht vollständig wirksam ist, wobei sich herausstellt, dass der Kork noch nicht vollständig sterilisiert ist, führen wir eine weitere finale Behandlung nach Wahl **durch**, wobei wir thermische (Infrarot), chemische (Ethylenoxid) oder physikalische (Ultraschall) Behandlungen kombinieren;
h) Zur weiteren Sicherheit führen wir schließlich das Anblasen mittels Mikroinjektoren **durch**, welche kurz vor dem Start der Wicklungsphase des Korkblatts angeordnet werden und dazu fähig sind, die linsenförmigen Hohlräume (Lentizellen) vollständig zu reinigen, wobei mögliche Rückstände von "roten Pulvern" innerhalb der linsenförmigen Hohlräume (Lentizellen) beseitigt werden, wobei insbesondere die (TCA) 2-4-6-Trichloressigsäure eine Ablagerung bildet, die für den Geschmack von Kork hauptverantwortlich ist;
i) Abschluss der vorhergehenden Phase h) vor dem Start der Wicklungsphase **durch** ein Absaugen mittels Mikro-Aspiratoren, welche an beiden Seiten des Blattes mit dem Ziel angeordnet werden, mögliche Ablagerungen von "roten Pulvern" auf den Seiten und innerhalb der Hohlräume des Blattes abzusaugen;
j) Durchführen einer Kontrolle an dem vollständig sterilisierten Blatt, welches frei von jedem Mittel und/oder Kontaminierungssubstanzen ist, zum Bestimmen seiner physikalischen Eigenschaften, um die Wickelspannung derart, dass die gewünschten Parameter einer Dichte, einer Kompressibilität, einer Porosität, einer Gaspermeabilität des resultierenden Verschlusses erreicht werden, und bereit für eine Herstellungsphase des Verschlusses einzustellen, und Vorbereiten des Blattes, um spiralförmig auf sich selbst oder auf einen aus Naturkork geeignet geformten Kern gewickelt zur werden, auf welchen sich das Korkblatt selbst als eine Spirale ohne einen Zusatz von Klebstoff oder Heißversiegelungsmaterialien wickelt;
l) Finale Kalibrierung, Trimmung, Schmierung des Endproduktes **durch** eine Verdampfung von Glycerin, um den Einsatz des Verschlusses in den Hals der Flasche zu begünstigen;
m) Abscherung des Blattes und Rückaufwickeln für die Herstellung der anschließenden Charge von Verschlüssen;
wobei das Verfahren **dadurch** gekennzeichnet ist, dass:
k) in einem Bereich mit gesteuerter/geregelter Umgebung, ohne eine Intervention äußerer Leime oder Klebstoffe, bei mehr als 40% Feuchtigkeit und bei einer Temperatur im Bereich zwischen 60 °C und 80 °C, wir die Freisetzung der wachsartigen Substanzen auf natürliche Art und Weise erhalten, welche in dem Kork enthalten sind (Ceroxid und Friedelin, welche etwa 5% der Zellwand des Korks bilden), die als ein natürlicher Klebstoff wirken, wobei infolgedessen das Selbstkleben **durch** ein Katalysieren der Substanzen der Korkblätter hergestellt wird, welche **durch** spiralförmiges Selbstaufwickeln **durch** ein Extrahieren und ein Transformieren der in diesen selbst enthaltenen wachsartigen Klebstoffsubstanzen an sich selbst kleben;
und, dass:
in einer Umgebung unter einstellbarem Druck von 4 bis 30 bar, wir die Platte oder das Blatt von Kork **durch** ein Einspannen seiner Enden dazu zwingen, sich auf sich selbst zu wickeln, wobei sie/es selbst klebt und sich selbst verdichtet, wobei bei einem Zurückkommen zu dem atmosphärischen Druck der Kork seine initialen natürlichen Abmessungen wiedererlangt, wobei dann die gewünschte Kompaktheit sowie die Luftdichtigkeit gewährleistet werden.

2. Verfahren zur Produktion und Herstellung von Verschlüssen aus spiralförmig gewickeltem Korkblatt nach Anspruch 1, **gekennzeichnet durch** die Tatsache, dass, **durch** ein Ausnutzen der Betriebsbedingungen bei einem Vakuumbetrieb, welche an die vorgegebenen Bedingungen der Feuchtigkeit und der Temperatur des Korkblatts einstellbar sind, wir das Blatt dazu zwingen, sich **durch** die Zentripetalkraft bis zu der Erstellung des Verschlusses auf sich selbst aufzuwickeln.

3. Verfahren zur Produktion und Herstellung von Verschlüssen aus spiralförmig gewickeltem Korkblatt nach einem der Ansprüche 1 bis 2, **gekennzeichnet durch** die Tatsache, dass das Blatt vorläufig **durch** ein Rollen, radial in Bezug auf die Wachstumsrichtung der Rinde, der Position in Bezug auf den Radius erhalten wird, welche die Eigenschaften einer Dichte und einer Porosität des Korks bestimmt, Eigenschaften, die hauptsächlich die Qualität des Verschlusses bestimmen, wobei es ermöglicht wird, Verschlüsse von höchster Qualität mit einer gleichmäßigen Dichte, einem gleichmäßigen Elastizitätsmodul und einer gleichmäßigen Porosität, wie gewünscht, zu erhalten.

4. Verfahren zur Produktion und Herstellung von Verschlüssen aus spiralförmig gewickeltem Korkblatt nach Anspruch 3, **gekennzeichnet durch** die Tatsache, dass der Kern zum Spiralwickeln konisch oder für die bestimmte Verwendung geeignet geformt ist.

5. Verfahren zur Produktion und Herstellung von Verschlüssen aus spiralförmig gewickeltem Korkblatt nach einem der Ansprüche 1 bis 4, durch eine anschließende Behandlung der erhaltenen Rolle oder des Verschlusses, welcher durch Schneiden der Rolle in einer gesteuerten/ geregelten Umgebung erhalten wird, wobei demzufolge eine Verdichtung und ein Selbstkleben verstärkt werden.

6. Verschluss aus spiralförmig gewickeltem Korkblatt, welcher gemäß dem in einem der Ansprüche 1 bis 5 beschriebenen Verfahren hergestellt ist, **gekennzeichnet durch** die Tatsache, dass dieser frei von jeglicher Synthetik- oder Kunststoffkomponente und völlig frei von jeglicher chemischer oder nicht natürlicher Klebstoffsubstanz ist, mit vorgegeben Eigenschaften von Abmessungen, einer Elastizität, einer Kompressibilität, einer Dichte, einer Porosität, einer Gaspermeabilität.

## Revendications

1. Procédé pour la production et fabrication de bouchons en feuille de liège enroulée en spirale, **caractérisé en ce qu'**il comprend les opérations suivantes, constituées des phases par la présente décrites :
a) première phase de nettoyage de la matière première par ébullition de 60 à 90 minutes
b) seconde ébullition ;
c) préparation de la feuille en commençant à partir de la planche de liège après l'élimination du ventre et du dos qui est de la partie en contact avec le tronc de l'arbre et de la partie superficielle à l'extérieur de l'écorce la plus externe ;
d) traitement dans la chambre hyperbare, sous des conditions de température s'étendant entre 100°C et 150°C, l'humidité et la durée variables s'étendant respectivement entre 60 % et 90 % et entre 1 et 24 heures, selon la quantité d'agents contaminants, tels que parasites, moisissures, mycètes et d'acide 2-4-6-trichloroacétique (TCA), de chloroanisole produit par le métabolisme du champignon *Armillaria mellea*, et des substances solubles dans l'eau telles que les sels minéraux et les tanins ;
e) analyse de vérification ultérieure du résultat obtenu par le traitement ci-dessus décrit au point d) ;
f) si les analyses suite au traitement décrit au point e) résultent **en ce que** le liège n'est pas complètement stérilisé, nous répétons un traitement additionnel dans une chambre hyperbare avec l'addition de dioxyde de carbone, la substance connue pour ses propriétés antibactériennes et fongicides, et/ou en présence d'addition de co-solvants ;
g) dans le cas où le traitement selon le point précédent f) ne s'avère à nouveau pas entièrement efficace mettant en relief que le liège n'est encore pas entièrement stérilisé, nous effectuons un traitement final supplémentaire comme la combinaison des traitements thermique (infrarouge), chimique (oxyde d'éthylène) ou physique (ultrasons) ;
h) pour la sécurité supplémentaire nous effectuons finalement le soufflage à l'aide de micro-injecteurs disposés juste avant le début de la phase d'enroulement de la feuille de liège, capable de nettoyer entièrement les creux lenticulaires (lenticelles) éliminant les résidus possibles de « poudres rouges » à l'intérieur desdits creux lenticulaires (lenticelles), où en particulier l'acide 2-4-6-trichloroacétique (TCA) se dépose, responsable principal du goût du liège ;
i) achèvement de la phase précédente h) avant le début de la phase d'enroulement, par aspiration à l'aide de micro-aspirateurs disposés sur les deux côtés de la feuille dans le but d'évacuer les résidus possibles de « poudres rouges » sur lesdits côtés et à l'intérieur des creux de ladite feuille ;
j) exécution d'une vérification de la feuille complétement stérilisée, exempte de chaque agent et/ou substance contaminant-e pour déterminer ses caractéristiques physiques pour définir la tension d'enroulement, pour atteindre les paramètres souhaités de densité, de compressibilité, de porosité, de perméabilité aux gaz du bouchon résultant et prête pour la phase de fabrication du bouchon, et la préparation de ladite feuille pour l'enroulement en spirale sur elle-même ou sur un coeur convenablement en forme de liège naturel sur lequel ladite feuille de liège s'enroule elle-même en spirale sans l'addition d'adhésif ni de matériaux d'étanchéité contre la chaleur ;
l) calibrage final, coupage, lubrification du produit final par la vaporisation de glycérine, pour favoriser l'insertion du bouchon dans le goulot de la bouteille ;
m) cisaillement de la feuille et ré-enroulement pour la fabrication du lot ultérieur de bouchons ;
ledit procédé étant **caractérisé en ce que** :
k) dans une zone d'environnement contrôlé, sans l'intervention de colles ni d'adhésifs externes, à plus de 40 % d'humidité et à une température s'étendant entre 60°C et 80°C, nous obtenons la libération de substances cireuses naturellement contenues dans le liège (oxyde cérique et de friedelane réalisant autour de 5 % de la paroi cellulaire du liège), qui agissent comme adhésif naturel produisant par conséquent l'auto-adhésion par catalyse desdites substances des feuilles de liège qui se collent par auto-enroulement en spirale sur elles-mêmes par l'extraction et la transformation desdites substances adhésives cireuses contenues en elles-mêmes ;
et **en ce que** :
dans un environnement sous pression ajustable, de 4 à 30 bars, nous forçons à travers le passage de ses extrémités la couche ou la feuille de liège pour l'enrouler sur elle-même par adhésion et en la compactant elle-même, en revenant à la pression atmosphérique, le liège retrouve ses dimensions naturelles initiales, puis en s'assurant de la compacité et de l'étanchéité à l'air souhaitées.

2. Procédé de production et de fabrication de bouchons de feuille de liège enroulée en spirale, selon la revendication 1, **caractérisé par le fait qu'**en exploitant les conditions de travail par opération sous vide ajustable aux conditions définies d'humidité et de température de la feuille de liège, nous forçons ladite feuille par force centripète à s'enrouler sur elle-même jusqu'à la construction du bouchon.

3. Procédé de production et de fabrication de bouchons de feuille de liège enroulée en spirale, selon l'une quelconque des revendications 1 à 2, **caractérisé par le fait que** la feuille est obtenue de manière préliminaire par enroulement, radialement par rapport au sens de croissance du liège, la position par rapport au rayon déterminant les caractéristiques de densité et de porosité du liège, les caractéristiques qui déterminent principalement la qualité des bouchons, permettant d'obtenir des bouchons d'une qualité supérieure avec une densité uniforme, un module d'élasticité et une porosité prédéfinis comme vous l'aimez.

4. Procédé de production et de fabrication de bouchons de feuille de liège enroulée en spirale, selon la revendication 3, **caractérisé par le fait que** le coeur enroulé en spirale est conique ou mis en forme de manière appropriée à l'utilisation prévue.

5. Procédé de production et de fabrication de bouchons de feuille de liège enroulée en spirale, selon l'une quelconque des revendications 1 à 4, par traitement ultérieur du rouleau obtenu ou des bouchons obtenus par coupe dudit rouleau sous environnement contrôlé en renforçant leur consolidation et leur auto-adhésion.

6. Bouchon de feuille de liège enroulée en spirale, fabriqué selon le procédé décrit selon l'une quelconque des revendications 1 à 5, **caractérisé par le fait qu'**il est exempt de quelconque composant synthétique ou plastique et entièrement exempt de quelconque substance adhésive chimique ou non d'origine naturelle, avec des caractéristiques prédéfinies de dimensions, d'élasticité, de compressibilité, de densité, de porosité, de perméabilité aux gaz.
